(19) Europäisches Patentamt
European Patent Office
Office européen des brevets

(11) Publication number : 0 350 842 B1

(12) EUROPEAN PATENT SPECIFICATION

(45) Date of publication of patent specification : 11.11.92 Bulletin 92/46

(51) Int. Cl.$^5$ : C09K 19/32, C07C 69/33, C07C 69/92

(21) Application number : 89112598.1

(22) Date of filing : 10.07.89

# (54) Pyramidal liquid crystals.

(30) Priority : 11.07.88 IT 2130888

(43) Date of publication of application : 17.01.90 Bulletin 90/03

(45) Publication of the grant of the patent : 11.11.92 Bulletin 92/46

(84) Designated Contracting States : CH DE ES FR GB LI NL SE

(56) References cited : EP-A- 0 300 800

(73) Proprietor : MONTEDISON S.p.A.
31, Foro Buonaparte
I-20100 Milan (IT)

(72) Inventor : Dalcanale, Enrico
28, via Turbigo
I-28067 Pernate Novara (IT)
Inventor : Bonsignore, Stefano
8, via Bergamo
I-28100 Novara (IT)
Inventor : Cometti, Giuseppe
14, via Crocetta
I-28048 Verbania-Pallanza Novara (IT)
Inventor : Du Vosel, Annick
2, Cascina Molinaccio
I-28100 Caltignaga, Novara (IT)

(74) Representative : Barz, Peter, Dr. et al
Patentanwälte Dipl.-Ing. G. Dannenberg Dr. P. Weinhold, Dr. D. Gudel Dipl.-Ing. S. Schubert, Dr. P. Barz Siegfriedstrasse 8
W-8000 München 40 (DE)

EP 0 350 842 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to liquid crystalline macrocyclic tetramers.

More particularly, the present invention relates to thermotropic dodeca-substituted macrocyclic tetramers which show mesophases of the pyramidal type.

The term "pyramidal mesophase", as used in the present description and in the claims, denotes a two-dimensional structure wherein the molecules are arranged in the form of columns. The molecules making up the columns are formed by a central nucleus of pyramidal shape with twelve substituent chains symmetrically distributed around the base (centre) of the pyramid. In such a column, the central nuclei are piled on each other and show the same orientation.

This type of monophase is described, for instance, in Journal de Physique, 47 (1986), page 351 and in Zeitschrift für Naturforschung, 40A (1985), page 149.

Liquid crystals and their use as electronic components (for example, in the optoelectronic field), for the non-destructive analysis of materials or in medical diagnosis and, more generally, as "displays", have been known for many years and are described in many publications such as, for example, US-A-4 430 650.

In US-A-4 619 788, hexa-substituted derivatives of tribenzocyclononatriene, showing monophases of the pyramidal type, are described.

These are molecules provided with a rigid central nucleus having a pyramidal structure wherein the chains of the substituents are symmetrically linked to the base of the pyramid.

These compounds have an electric dipolar moment which makes them particularly suitable for use as "memory devices" or as displays in optoelectronics.

It has now been found that dodeca-substituted derivatives of the cyclic tetramer, which are obtainable by acid-catalyzed condensation of pyrogallol and an aliphatic (alkyl) aldehyde and wherein the substituents are alkanoyl chains, are stable products, anisotropic in the molten state, and show monophases of the pyramidal type.

Therefore, object of the present invention are thermotropic dodeca-substituted macrocyclic tetramers of the general formula:

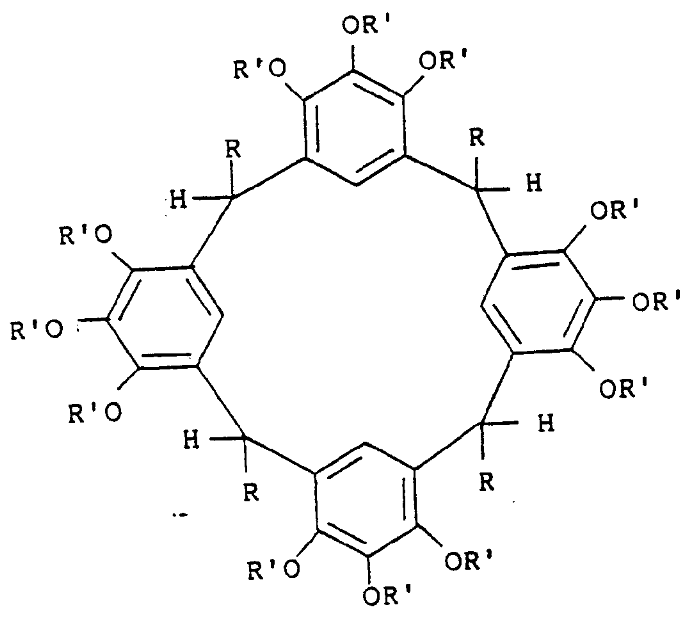

(I)

wherein the groups R are alkyl radicals containing 1 or 2 carbon atoms and the groups R′, the same or different from each other, represent a radical of the general formula:

$$-CO-C_nH_{2n+1} \qquad (II)$$

wherein n is an integer greater than or equal to 12.

Among the compounds of general formula (I) those, wherein n is an integer of from 12 to 30, are the preferred ones. Although the groups R′ can be the same or different from each other, compounds wherein all of the radicals R′ have the same meaning are preferred.

In the thermotropic macrocylic tetramers of the present invention the groups R are arranged in axial position, as shown in Fig. 1, which represents a perspective view of the molecule. This type of structure tends to

form hexagonal columns of pyramidal shape in the mesophase.

The tetramers of general formula (I) usually have melting temperatures of from about 30 to about 100°C.

The clearing points of the molten products generally range from about 60 to about 120°C.

The pyramidal-column liquid crystalline structure can be inferred from differential scanning calorimetry (DSC), analysis with a polarized light optical microscope and X-ray diffraction. In particular, for all of the mesogens of the present class, a mesophase of the "broken fan" type is observed under the microscope, said mesophase being typical for highly organized column-shaped liquid crystals. By slow cooling (starting from the isotropic state), an omeotropic organization of the mesophase is obtained, wherein the columns are oriented perpendicularly to the surface of the slide. In contrast thereto, by allowing the mesophase to slide on a rigid support, a planar arrangement of the columns with respect to the surface of the slide is observed.

The X-ray diffraction of the non-oriented mesophase shows two diffraction rings: The first one is very wide with great angles, characteristic for the molten aliphatic chains; The second one is intense and narrow with small angles, representing the two-dimensional organization in the plane.

Said observations allow the mesophase to be defined as hexagonal column-shaped and belonging to the symmetry group ($D_{ho}$).

In Fig. 2 the transition temperatures of the different phases of a macrocyclic tetramer of the general formula (I), wherein R is $CH_3$ are shown for exemplary purposes.

In said figure, the temperatures are given on the ordinate, whereas the values of n of the radical R′ are indicated on the abscissa. The letters I, P and K denote the isotropic phase, the pyramidal liquid crystalline phase and the crystalline phase, respectively. In particular, in Fig. 2, the circles represent the "clearing points", whereas squares represent the transition crystal-pyramidal mesophase.

The thermotropic macrocylic tetramers of the present invention may be prepared by reacting a macrocyclic tetramer of the general formula:

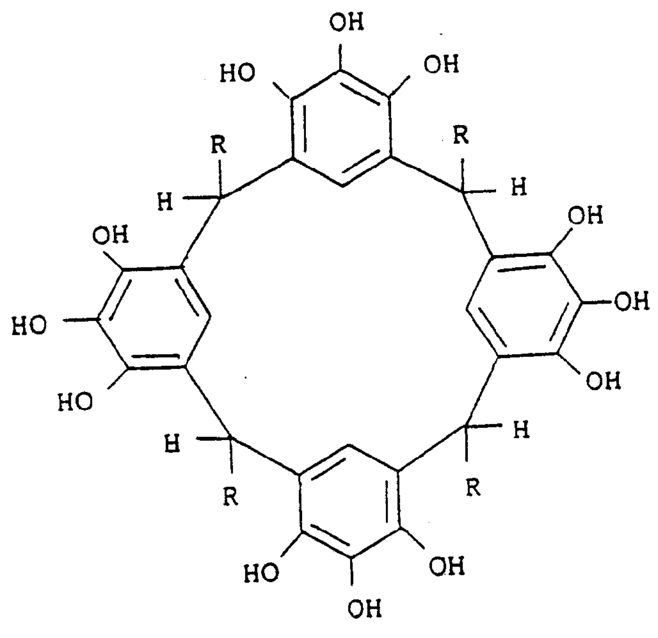

(III)

wherein R is as defined above, with acyl halides of the general formula R′-X, wherein X represents a halogen atom, preferably chlorine, and R′ has the meanings given above. Examples of suitable acyl halides are: myristoyl chloride, palmitoyl chloride, stearoyl chloride, tridecanoyl chloride, etc.

The above reaction may, for instance, be carried out as a conventional bulk esterification under normal pressure and at temperatures of from about +50°C to about +200°C.

The macrocyclic tetramers of general formula (III) may be obtained by acid-catalyzed condensation at room temperature of pyrogallol and the acetal of an aliphatic (alkyl) aldehyde in a polar solvent, preferably an alcohol such as methanol or ethanol. The catalyst may, for instance, be selected from hydrochloric acid, sulfuric acid, phosphoric acid, para-toluene sulfonic acid, etc. These catalysts are generally used in amounts of from 1 to 20% by weight, based on the total amount of the reagents.

The substrates of general formula (III) may, for instance, be prepared according to the following reaction scheme:

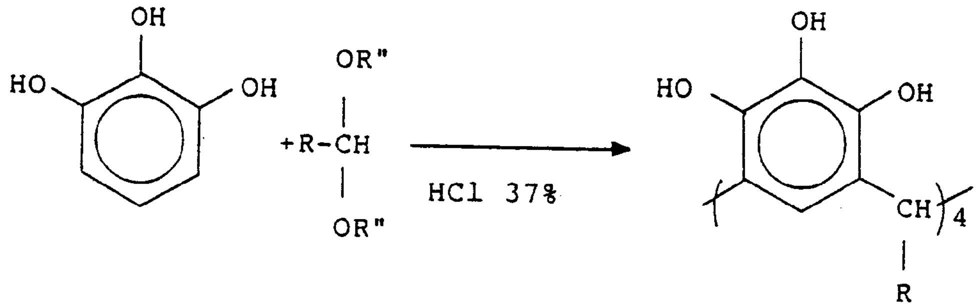


wherein R is an alkyl radical containing 1 or 2 carbon atoms and R″ is an alkyl, cycloalkyl, aryl or alkylaryl radical containing from 1 to 30 carbon atoms. Preferably, the groups R″ are the same. Examples of radicals R″ include linear or branched alkyl groups having 1 to 30, preferably 1 to 12 and, particularly, 1 to 6 carbon atoms (for example, methyl, ethyl, n- and i-propyl and butyl); cycloalkyl groups having 3 to 8 carbon atoms (for example, cyclopentyl and cyclohexyl); aryl groups having 6 to 14 carbon atoms (for example, phenyl, naphthyl and biphenylyl); alkylaryl groups having 7 to 15 carbon atoms. Furthermore, the groups R″ may be combined to form an alkylene group of preferably 2 or 3 carbon atoms. Particularly preferred are alkyl groups R″ as defined above.

With the above process, only one of the many possible configurational isomers is obtained, namely the isomer called "crown" (Fig. 3), wherein all of the groups R are in the axial position.

Because of their pyramidal shape, the macrocyclic tetramers of the present invention have an intrinsic electric dipole moment, oriented in the direction of the symmetry axis $C_4$ of the molecules. This characteristic, together with the arrangement of the pyramidal mesophase in columns wherein the central nuclei are piled on each other and all show the same orientation, makes the columns polar. The polar axis coincides with the symmetry axis of the column. This is why the pyramidal mesophase formed by the macrocyclic tetramers of the present invention can be ferroelectric if all of the columns assume the same direction of polarization at macroscopic level (see Fig. 4). Generally, the column-shaped mesophases of the hexagonal type tend to favour the ferroelectric orientation over the anti-ferroelectric orientation. Furthermore, the same result can be obtained by orienting the columns under the influence of an external electric field.

The above properties make the thermotropic macrocyclic tetramers of the present invention particularly suitable for use in "memory devices", in optoelectronic displays, in phototonics as components for "non-linear optical devices" and, in general, in all fields wherein liquid crystals can be employed.

The following examples are given in order to illustrate the present invention without, however, limiting it.

EXAMPLE 1

Preparation of 3.4.5.10.11.12.17.18.19.24.25.26-dodecahydroxy-c-1,c-8,c-15,c-22-tetramethyl-[$1_4$]metacyclophane

12.61 g (0.1 mol) of pyrogallol were dissolved, under argon, in 50 ml of ethanol and 10 ml of 37% HCl.

The solution was cooled to 0°C by means of an ice bath and 14.22 ml of 1,1-diethoxyethane were added dropwise within 1 hour. Thereafter, the solution was kept at room temperature under agitation fo 48 hours, whereupon it was heated to reflux for 6 hours. The resulting mixture was cooled to room temperature, water was added thereto and the formed precipitate was filtered off and repeatedly washed until neutral. The product was dried under vacuum (1 x $10^{-3}$ torr/25°C) to afford 11.0 g of pure product (yield 72%).

Mass ($DCI^+$): $MH^+$=609

$^1$H-NMR (DMSO-$d_6$): δ 1.55 (d, 12H, J=7.5 Hz, $CH_3$); 4.56 (q, 4H, J=7.5 Hz, CH); 6.79 (s, 4H, Ar-H); 8.02 (bs, 4H, OH); 8.22 (s, 8H, OH).

Elemental analysis for $C_{32}H_{32}O_{32}$:

| | |
|---|---|
| Calculated: | C= 63.15% H = 5.30% |
| Found: | C = 63.01% H = 5.43% |

EXAMPLE 2

Preparation of 3.4.5.10.11.12.17.18.19.24.25.26-dodecatridecanoyloxy-c-1,c-8,c-15,c-22-tetramethyl-[$1_4$]metacyclophane

0.609 g (1 mmol) of the tetramer obtained in example 1 and 10.1 ml (40 mmol) of tridecanoyl chloride were heated for 10 hours at 140°C under agitation. After completion of the reaction, the excess chloride was distilled off under vacuum ($10^{-2}$ torr). The residue was dissolved in methylene chloride and the resulting solution was first extracted with 0.1N NaOH, then washed with water until neutral and, thereupon, dried over sodium sulfate. The solvent was evaporated and the crude product thus obtained was purified on a silica gel column using methylene chloride/hexane (8:2) as eluent. 1.72 g of pure product were obtained (yield 58%).

Mass ($DCI^-$): $M^-$=2960

$^1$H-NMR ($CDCl_3$): δ 0.89 [t, 36H, $(CH_2)_n$-$\underline{CH_3}$]; 1.30 [bs, 216H, $(CH_2)_9$]; 1.45 (d, 12H, J=7.4 Hz, CH-$\underline{CH_3}$); 1.55 (bm, 12H, CO-$CH_2$-$\underline{CH_2}$); 1.71 (bm, 12H, CO-$CH_2$-$\underline{CH_2}$′); 2.28 (bm, 12H, CO-$CH_2$); 2.50 (bm, 12H, CO-$CH_2$′); 4.22 (q, 4H, J=7.4 Hz, CH); 6.03 (s, 2H, Ar-H); 7.31 (s, 2H, Ar-H′).

Elemental analysis for $C_{188}H_{320}O_{24}$:

| | |
|---|---|
| Calculated: | C = 76.17% H = 10.88% |
| Found: | C = 76.17% H = 11.08% |

EXAMPLE 3 to 5

By following the procedure of example 2, using myristoyl chloride, palmitoyl chloride and stearoyl chloride respectively, the following were obtained:

3.4.5.10.11.12.17.18.19.24.25.26-dodecatetradecanoyloxy-c-1,c-8,c-15,c-22-tetramethyl-[$1_4$]metacyclophane (yield 54% after purification on a silica gel column, using methylene chloride/hexane (9:1) as eluent.

Mass ($DCI^-$): $M^-$= 3128.

$^1$H-NMR ($CDCl_3$): δ 0.89 [t, 36H, $(CH_2)_n$-$\underline{CH_3}$]; 1.30 [bs, 240H, $(CH_2)_{10}$]; 1.47 (d, 12H, J = 7.4 Hz, CH-$\underline{CH_3}$); 1.53 (bm, 12H, CO-$\underline{CH_2}$); 1.72 (bm, 12H, CO-$CH_2$-$\underline{CH_2}$′); 2.29 (bm, 12H, CO-$CH_2$); 2.51 (bm, 12H, CO-$CH_2$′); 4.22 (q, 4H, J=7.4 Hz, CH); 6.02 (s, 2H, Ar-H); 7.31 (s, 2H, Ar-H′).

Elemental analysis for $C_{200}H_{344}O_{24}$

| | |
|---|---|
| Calculated: | C = 76.67% H = 11.07% |
| Found: | C = 76.71% H = 11.26% ) |

3.4.5.10.11.12.17.18.19.24.25.26-dodeca-hexadecanoyloxy-c-1,c-8,c-15,c-22-tetramethyl-[$1_4$]metacyclophane (yield 50% after two crystallizations from methylene chloride/ethyl ether.

Mass ($DCI^-$): $M^-$=3464

$^1$H-NMR ($CDCl_3$): δ 0.89 [t, 36H, $(CH_2)_n$-$\underline{CH_3}$]; 1.29 [bs, 288H, $(CH_2)_{12}$]; 1.45 (d, 12H, J=7.4 Hz, CH-$\underline{CH_3}$); 1.49-1.80 (m, 24H, CO-$CH_2$-$\underline{CH_2}$); 2.28 (bm, 12H, CO-$CH_2$); 2.51 (bm, 12H, CO-$CH_2$′); 4.22 (q, 4H, J=7.4 Hz, CH); 6.01 (s, 2H, Ar-H); 7.30 (s, 2H, Ar-H′).

Elemental analysis for $C_{224}H_{392}O_{24}$:

| | |
|---|---|
| Calculated: | C = 77.55% H = 11.39% |
| Found: | C = 77.52% H = 11.58% ) |

3.4.5.10.11.12.17.18.19.24.25.26-dodeca-octadecanoyloxy-c-1,c-8,c-15,c-22-tetramethyl-[$l_4$]metacyclophane (yield 53% after two crystallizations from methylene chloride/ethyl ether.

Mass ($DCI^-$): $M^-$= 3800

$^1$H-NMR ($CDCl_3$): δ 0.89 [t, 36H, $(CH_2)_n$-$\underline{CH_3}$]; 1.30 [bs, 336H, $(CH_2)_{14}$]; 1.46 (d, 12H, J=7.4 Hz, CH-$\underline{CH_3}$); 1.70 (m, 24H, CO-$CH_2$-$\underline{CH_2}$); 2.30 (bm, 12H, CO-$CH_2$); 2.51 (bm, 12H, CO-$CH_2$′); 4.23 (q, 4H, J=7.4 Hz, CH); 6.03 (s, 2H, Ar-H); 7.30 (s, 2H, Ar-H′).

Elemental analysis for $C_{248}H_{440}O_{24}$:

| | |
|---|---|
| Calculated: | C = 78.26% H = 11.65% |

Found: C = 78.30% H = 11.70% )

EXAMPLES 6 and 7 (Comparative Examples)

By following the procedure of example 2, using decanoyl chloride and lauroyl chloride, respectively, two non-liquid crystalline products were obtained, namely:

3.4.5.10.11.12.17.18.19.24.25.26-dodeca-decanoyloxy-c-1,c-8,c-15,c-22-tetramethyl[$1_4$]metacyclophane (yield 52% after purification on a silica gel column, using methylene chloride/hexane (9:1) as eluent.

Mass (DCI$^-$): $M^-$=2456

$^1$H-NMR ($CDCl_3$): δ 0.89 [t, 36H, $(CH_2)_n$]; 1.25 (bs, 144H, $(CH_2)_6$]; 1.48 (d, 12H, J=7.4 Hz, CH-$\underline{CH_3}$); 1.52 (bm, 12H, CO-$CH_2$-$CH_2$); 1.71 (bm, 12H, CO-$CH_2$-$\underline{CH_2}$'); 2.30 (bm, 12H, CO-$CH_2$); 2.50 (bm, 12H, CO-$CH_2$'); 4.22 (q, 4H, J=7.4 Hz, CH); 6.02 (s, 2H, Ar-H); 7.30 (s, 2H, Ar-H').

Elemental analysis for $C_{152}H_{248}O_{24}$:

Calculated: C = 74.22% H = 10.16%
Found: C = 74.13% H = 10.45% )

3.4.5.10.11.12.17.18.19.24.25.26-dodeca-dodecanoyloxy-c-1,c-8,c-15,c-22-tetramethyl[$1_4$]metacyclophane (yield 58% after purification on a silica gel column, using methylene chloride/hexane (9:1) as eluent.

Mass (DCI$^-$): $M^-$=2792.

$^1$H-NMR ($CDCl_3$): δ 0.89 [t, 36H, $(CH_2)_n\underline{CH_3}$]; 1.24 [bs, 192H, $(CH_2)_8$]; 1.45 (d, 12H, J=7.4 Hz, CH-$\underline{CH_3}$); 1.54 (bm, 12H, CO-$CH_2$-$\underline{CH_2}$); 1.71 (bm, 12H, CO-$CH_2$-$\underline{CH_2}$'); 2.30 (bm, 12H, CO-$CH_2$); 2.52 (bm, 12H, CO-$CH_2$'); 4.22 (q, 4H, J=7.4 Hz, CH); 6.04 (s, 2H, Ar-H); 7.32 (s, 2H, Ar-H').

Elemental analysis for $C_{176}H_{296}O_{24}$:

Calculated: C = 75.60% H = 10.67%
Found: C = 75.69% H = 10.90% )

The following Table 1 lists the transition temperatures and the enthalpies of the products described in examples 2 to 7.

# TABLE 1

Transition temperatures (°C) and enthalpies (kJ/mol in parenthesis) of dodeca-substituted tetramers of general formula (I)

($R = CH_3$; $R' = CO\text{-}C_nH_{2n+1}$)

| n | Yield | K | | P | | I |
|---|---|---|---|---|---|---|
| 9 | 52% | * | | | 77 (12.6) | * |
| 11 | 58% | * | | | 71 (12.0) | * |
| 12 | 58% | * | 46 (118.6) | * | 70 (12.1) | * |
| 13 | 54% | * | 48 (185.5) | * | 67 (12.5) | * |
| 15 | 50% | * | 48 (78.0) | * | 61 (10.0) | * |
| 17 | 53% | * | 58 (87.0) | * | 68 (13.0) | * |

I = Isotropic phase
K = Crystalline phase
P = Liquid crystalline column-shaped phase of the pyramidal type
* = Observed phase

## Claims

1. Thermotropic dodeca-substituted macrocyclic tetramers of the general formula:

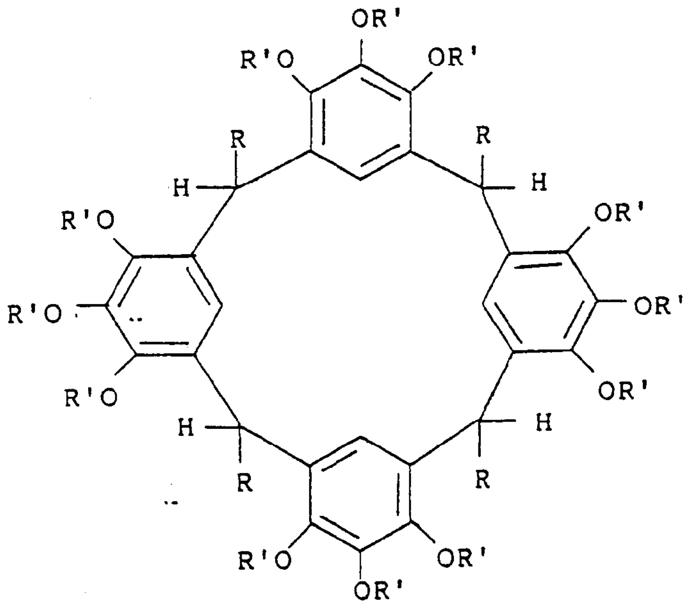

(I)

wherein the groups R are alkyl radicals containing 1 or 2 carbon atoms and the groups R′, the same or different from each other, represent a radical of the general formula

$$-CO-C_nH_{2n+1} \qquad (II)$$

wherein n is an integer greater than or equal to 12.

2. Tetramers according to claim 1, wherein n is an integer of from 12 to 30.

3. Tetramers according to claim 1 or 2, wherein all of the radicals R′ are the same.

4. Tetramers according to any one of the preceding claims having melting temperatures of from about 30°C to about 100°C and isotropization temperatures in the molten state (clearing points) of from about 60°C to about 120°C.

5. Tetramers according to any one of the preceding claims, wherein the monophase is of the hexagonal column shaped type with pyramidal arrangement.

6. Use of the tetramers according to any one of the preceding claims as components of "memory devices", in optoelectronic displays and in the field of phototonics as components of non-linear optical devices.

7. Process for the preparation of macrocyclic tetramers of the general formula:

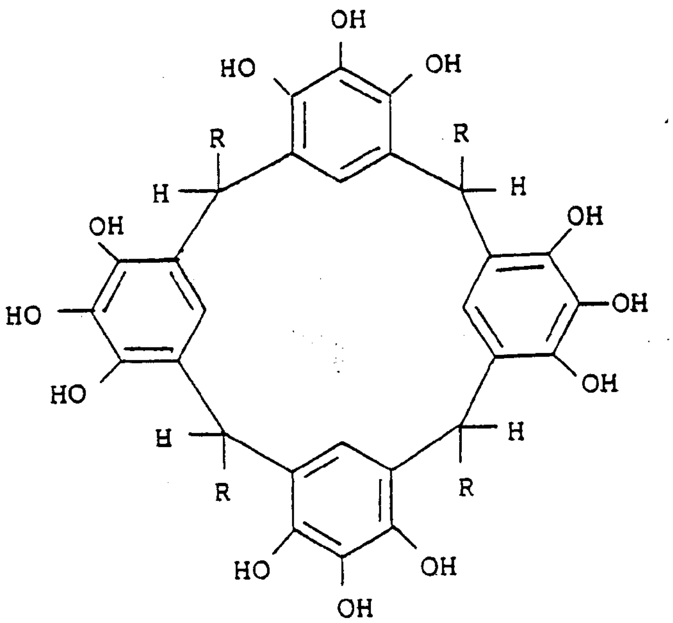

(III)

wherein R is defined as in claim 1, comprising the reaction, at room temperature, of pyrogallol and an acetal of an aliphatic aldehyde in the presence of an acidic catalyst and in a polar solvent, preferably an alcohol.

**8.** Process according to claim 7, wherein the acetal of the aliphatic aldehyde has the general formula:

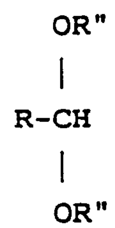

wherein R is a $C_1$-$C_2$ alkyl radical and R" is an alkyl, cycloalkyl, aryl or alkylaryl radical containing from 1 to 30 carbon atoms.

**9.** Process according to claim 7 or 8, wherein the catalyst is selected from hydrochloric acid, sulfonic acid, phosphoric acid and p-toluene sulfonic acid.

**Patentansprüche**

**1.** Thermotrope dodeca-substituierte makrocyclische Tetramere der allgemeinen Formel:

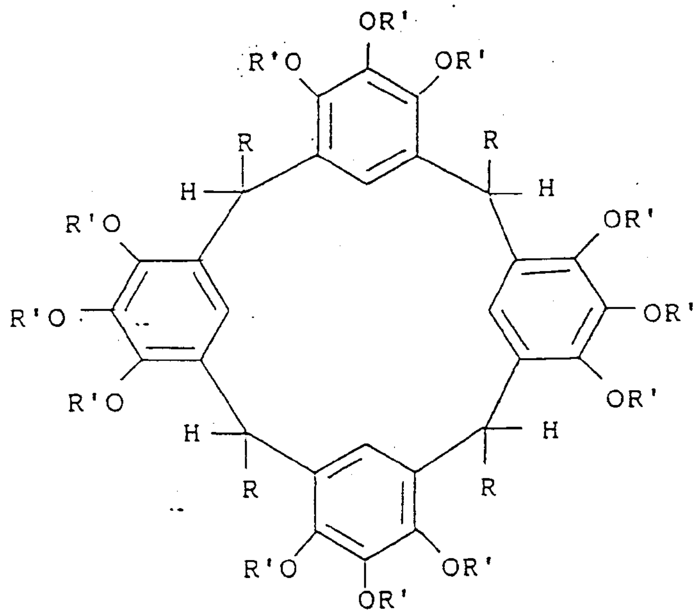

(I)

worin die Gruppen R Alkylradikale, die 1 oder 2 Kohlenstoffatome enthalten, sind und die Gruppen R′, gleich oder verschieden voneinander, einen Rest der allgemeinen Formel

$$-CO-C_nH_{2n+1} \qquad (II)$$

worin n eine ganze Zahl größer oder gleich 12 ist, darstellen

2. Tetramere gemäß Anspruch 1, worin n eine ganze Zahl von 12 bis 30 ist.

3. Tetramere gemäß Anspruch 1 oder 2, worin alle Reste R′ gleich sind.

4. Tetramere gemäß irgendeinem der vorangehenden Ansprüche mit Schmelztemperaturen von ungefähr 30°C bis ungefähr 100°C und Isotropisierungstemperaturen im geschmolzenen Zustand (Klarpunkte) von ungefähr 60°C bis ungefähr 120 °C.

5. Tetramere gemäß irgendeinem der vorangehenden Ansprüche, worin die Monophase vom hexagonalen säulenförmigen Typ mit pyramidaler Anordnung ist.

6. Verwendung der Tetrameren gemäß irgendeinem der vorangehenden Ansprüche als Komponenten von"Speichervorrichtungen", in optoelektronischen Anzeigen und auf dem Gebiet der Phototonik als Komponenten von nicht-linearen optischen Vorrichtungen.

7. Verfahren zur Herstellung von makrocyclischen Tetrameren der allgemeinen Formel:

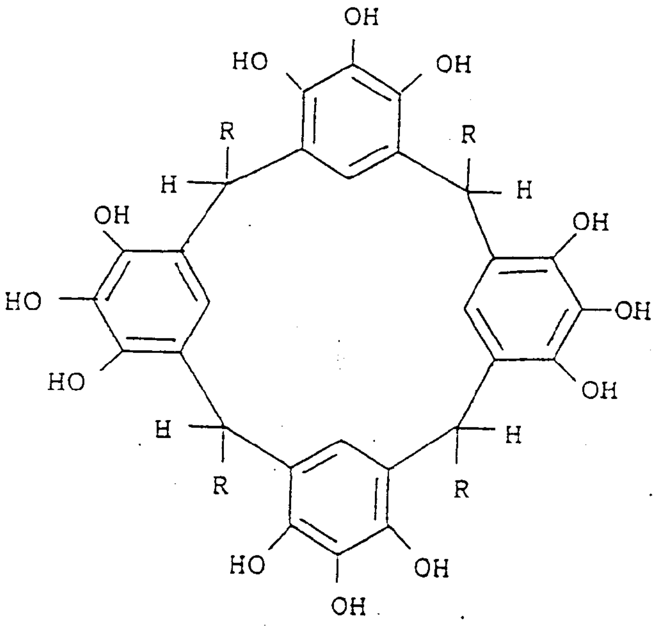

(III)

worin R wie in Anspruch 1 definiert ist, umfassend die Umsetzung, bei Raumtemperatur, von Pyrogallol und einem Acetal eines aliphatischen Aldehyds in Anwesenheit eines sauren Katalysators und in einem polaren Lösungsmittel, vorzugsweise einem Alkohol.

8. Verfahren nach Anspruch 7, worin das Acetal des aliphatischen Aldehyds die allgemeine Formel:

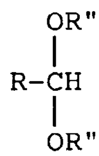

aufweist, worin R ein $C_1$-$C_2$ Alkylrest ist und R″ ein Alkyl-, Cycloalkyl-, Aryl- oder Alkylarylrest, der 1 bis 30 Kohlenstoffatome enthält, ist.

9. Verfahren nach Anspruch 7 oder 8, worin der Katalysator ausgewählt ist aus Chlorwasserstoffsäure, Sulfonsäure, Phosphorsäure und p-Toluolsulfonsäure.

## Revendications

1. Tétramères macrocycliques thermotropiques dodéca-substitués de formule générale:

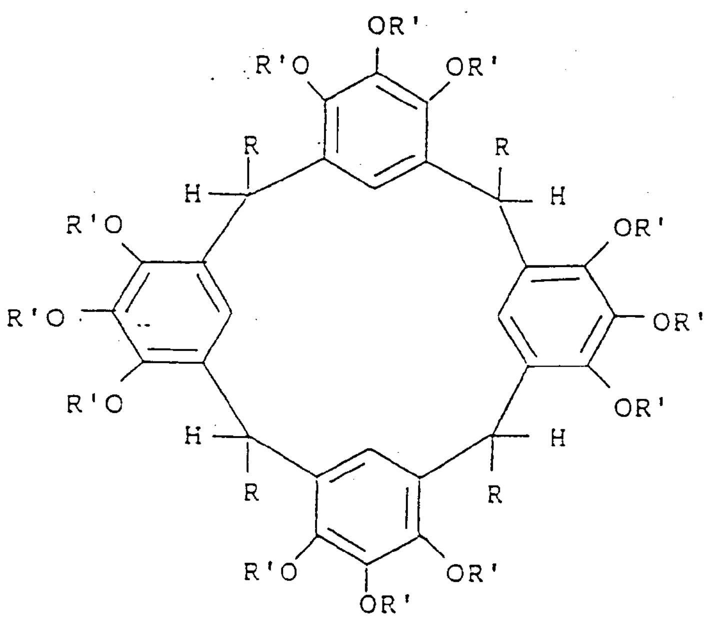

(I)

dans laquelle les groupes R sont des radicaux alkyle contenant 1 ou 2 atomes de carbone, et les groupes R′, identiques ou différents l'un de l'autre, représentent un radical de formule générale:

$$-Co\text{-}C_nH_{2n+1} \qquad (II)$$

dans laquelle n est un nombre entier supérieur ou égal à 12.

2. Tétramères selon la revendication 1, caractérisés en ce que n est un nombre entier compris entre 12 et 30.

3. Tétramères selon la revendication 1 ou 2, caractérisés en ce que tous les radicaux R′ sont identiques.

4. Tétramères selon l'une quelconque des revendications précédentes, dont les températures de fusion sont comprises entre environ 30 et environ 100°C et les températures d'isotropisation à l'état fondu (point de clarification) sont comprises entre environ 60 et environ 120°C.

5. Tétramères selon l'une quelconque des revendications précédentes, caractérisés en ce que la monophase est du type à conformation en colonnes hexagonales présentant une disposition pyramidale.

6. Utilisation des tétramères selon l'une quelconque des revendications précédentes comme composants de "dispositifs mémoires", dans des affichages optoélectroniques et dans le domaine de la phototonique comme composants de dispositifs optiques non linéaires.

7. Procédé de préparation de tétramères monocycliques de formule générale:

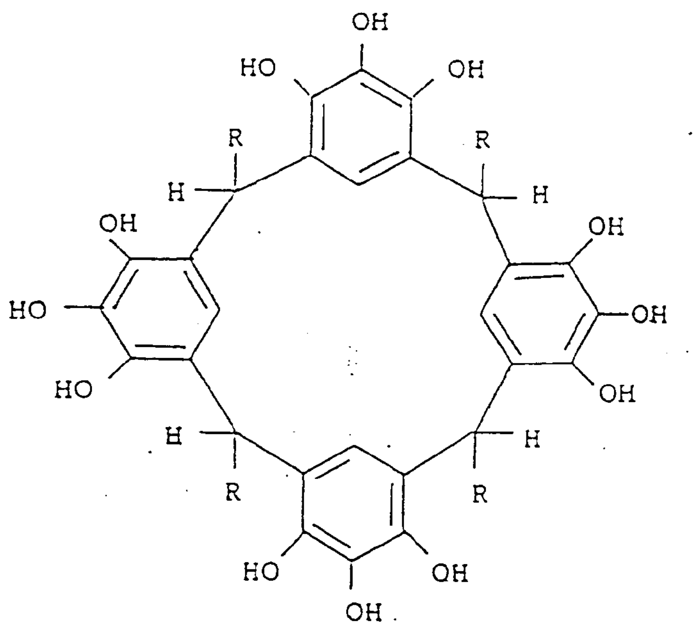

(III)

dans laquelle R est tel que défini dans la revendication 1, comprenant la réaction à la température ambiante du pyrogallol et d'un acétal d'un aldéhyde aliphatique en présence d'un catalyseur acide et dans un solvant polaire, de préférence un alcool.

**8.** Procédé selon la revendication 7, caractérisé en ce que l'acétal de l'aldéhyde aliphatique répond à la formule générale:

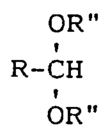

dans laquelle:

R est un radical alkyle en $C_1$ à $C_2$ et

R" est un radical alkyle, cycloalkyle, aryle ou alkyl-aryle contenant de 1 à 30 atomes de carbone.

**9.** Procédé selon la revendication 7 ou 8, caractérisé en ce que le catalyseur est choisi parmi l'acide chlorhydrique, l'acide sulfonique, l'acide phosphorique et l'acide p-toluènesulfonique.

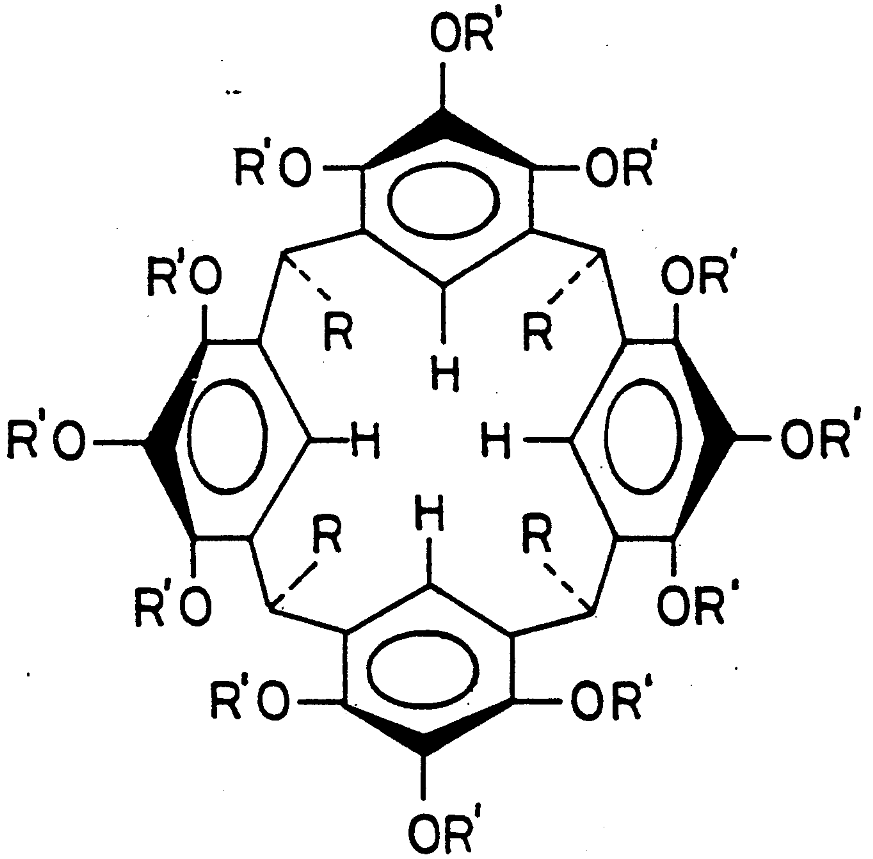

F I G. 1

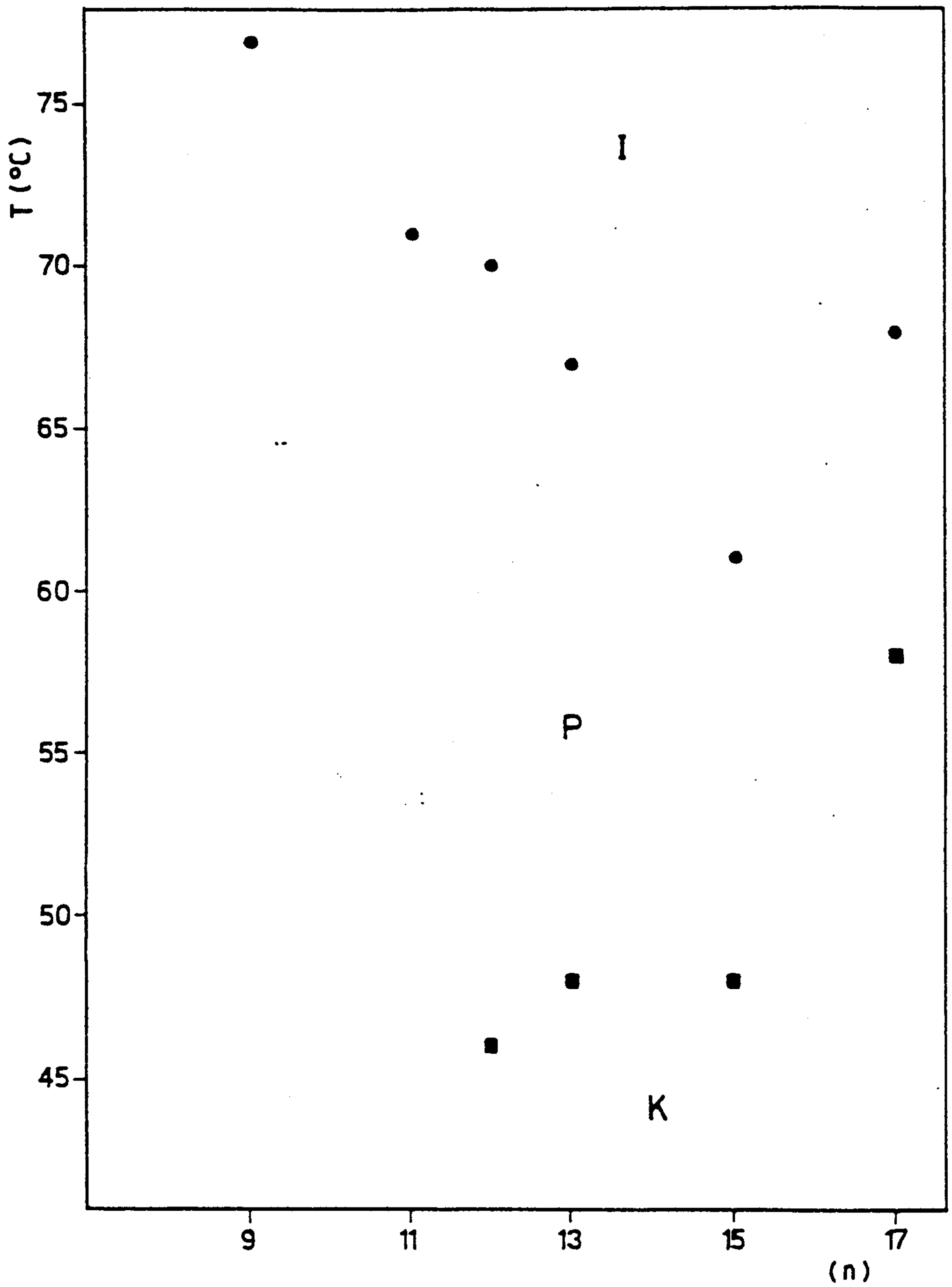


F I G. 2

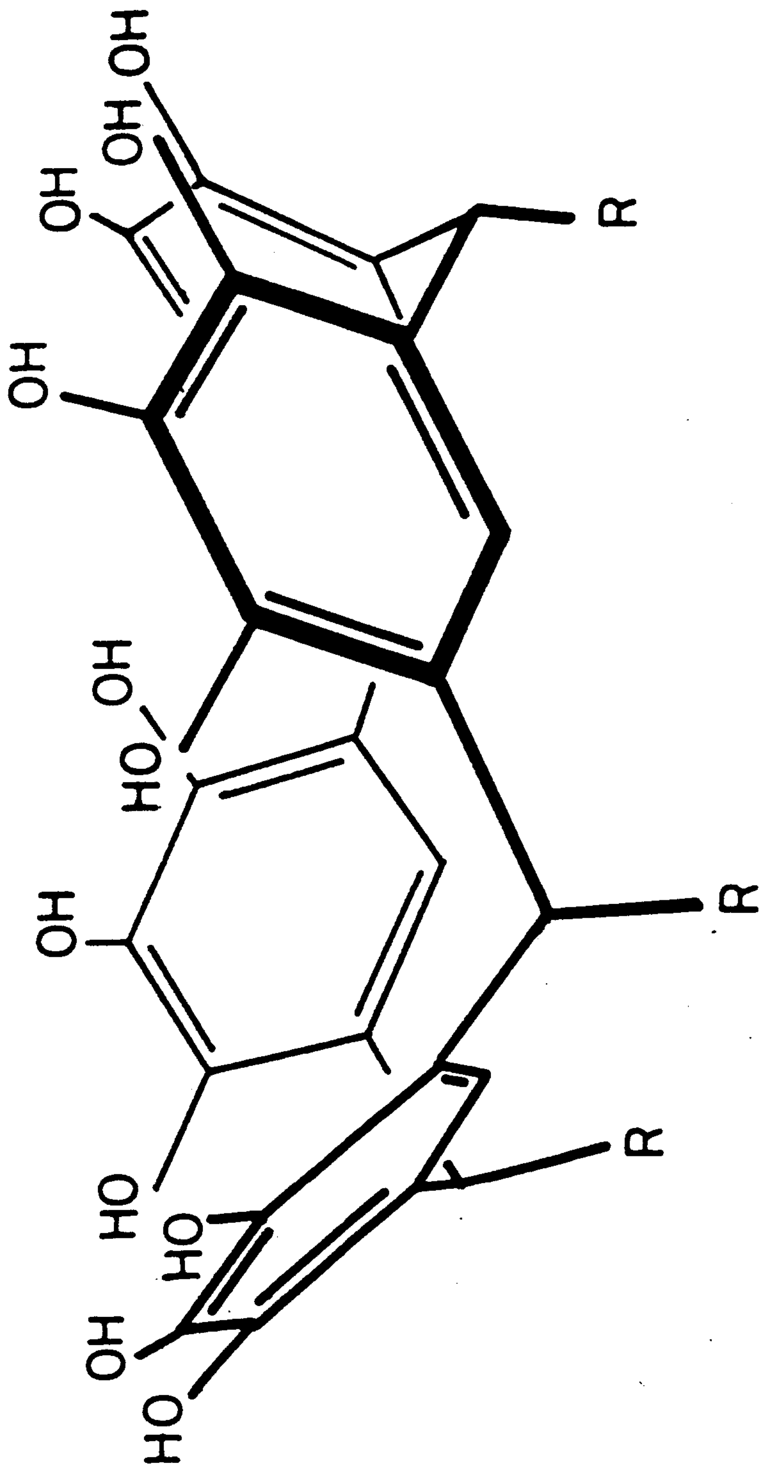

FIG. 3

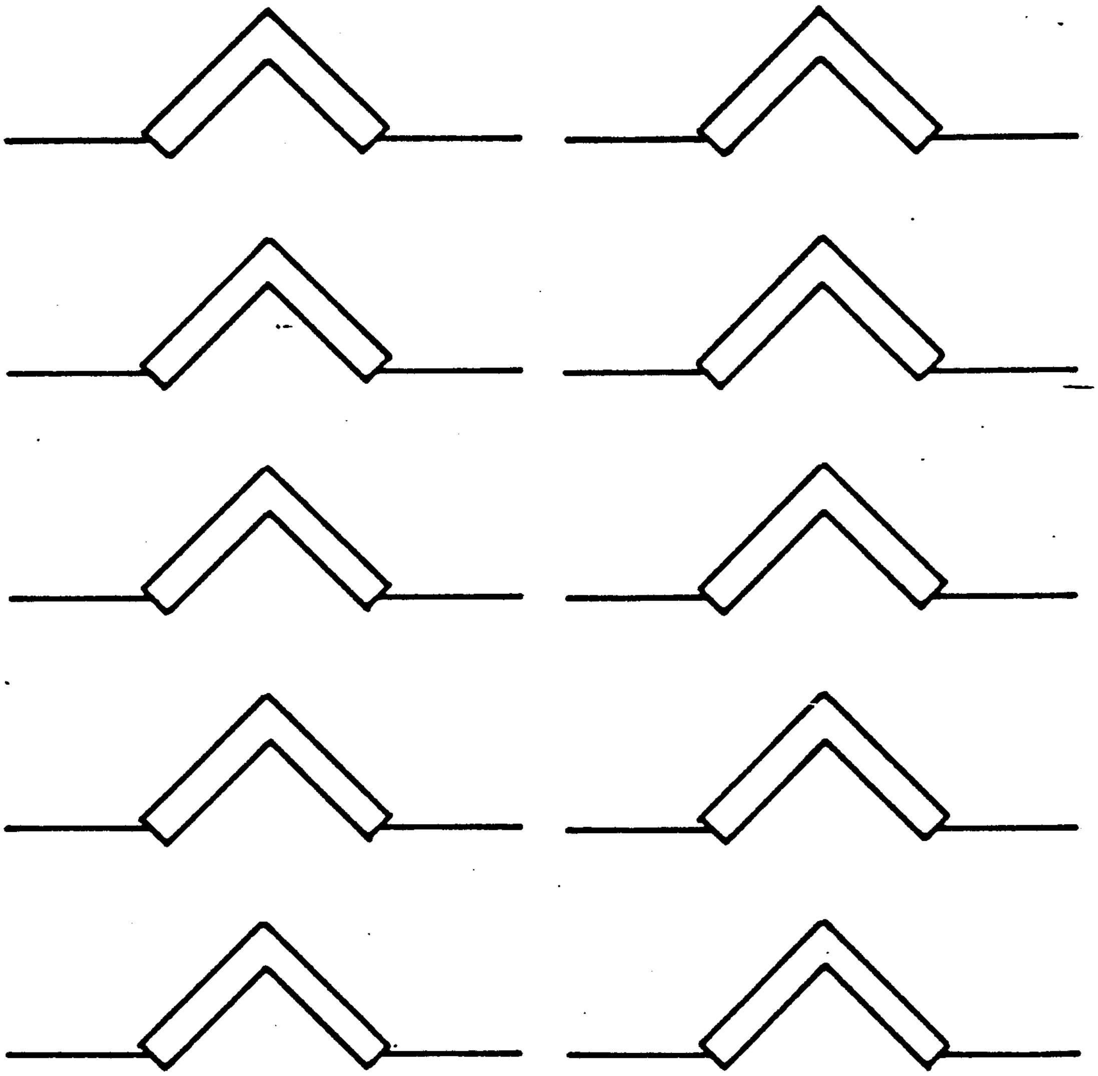

F I G. 4